# EUROPEAN PATENT SPECIFICATION

(11) **EP 0 802 963 B1**
(45) Date of publication and mention of the grant of the patent: **26.07.2006**
(21) Application number: 96942745.9
(22) Date of filing: 13.11.1996
(51) Int. Cl.: C10M 107/10, C10M 105/04, C10N 20/02, C10N 30/00

(54) **BIODEGRADABLE POLYALPHAOLEFIN FLUIDS AND FORMULATIONS CONTAINING THE FLUIDS**
BIOLOGISCH ABBAUBARE POLYALPHAOLEFINFLUIDE UND DIE FLUIDE ENTHALTENDE FORMULIERUNGEN
LIQUIDES BIODEGRADABLES CONSTITUES DE POLYALPHAOLEFINES, ET FORMULATIONS CONTENANT CES LIQUIDES

(30) Priority: 14.11.1995 GB 9523246
(43) Date of publication of application: 29.10.1997
(73) Proprietor: Innovene USA LLC, Lisle, Illinois 60532 (US)
(72) Inventor: BENDA, Rainer, B-1150 Brussels (BE); BULLEN, John, V., Berkshire RG12 3EJ (GB); PLOMER, Alan, J., B-1330 Rixenstart (BE)
(74) Representative: Hamer, Christopher K.
(86) International application number: PCT/US1996/018169
(87) International publication number: WO 1997/018280

(56) References cited:
- EP-A- 0 468 109
- EP-A- 0 558 835
- EP-A- 0 613 873
- US-A- 4 218 330

## Description

This invention relates generally to synthetic fluids prepared by oligomerizing linear α-olefins and more particularly to a synthetic fluid having improved biodegradability which is an oligomer of a mixture of C₁₂ and C₁₄ alpha-olefins.

Alpha-olefin oligomers, commonly referred to as polyalphaolefins or PAOs, and their use as hydraulic fluids and lubricants (synlubes) are well known. The oligomers are usually prepared from 1-decene for optimum properties, although mixtures of a-olefins have been used, as described, for example, in U.S. Patents 3,330,883 and 3,576,898. One advantage of the α-olefin fluids is that they are more biodegradable than mineral oil basestocks of like viscosity. This property is important from an environmental standpoint, especially in applications such as hydraulic fluids for earth- and water-moving equipment, automotive crankcase oils, heavy duty diesel oils, 2-stroke oils, automatic transmission fluids, and automotive gear oils. Biodegradability, however, tends to decrease as the viscosity of the fluids increases (higher oligomers). Surprisingly, we have found that oligomers made from certain C₁₂ and C₁₄ α-olefin mixtures have enhanced biodegradability compared to 1-decene based fluids.

EP 0 613 873 A2 discloses a process for preparing base materials that are suitable for lubricants, the process comprising catalytic oligomerisation of one or more C₈-C₂₀ alpha-olefins. The compositions referred to in the examples include C₁₂ and C₈ oligomers and mixtures thereof.

EP 0 558 835 discloses unhydrogenated oligomers of C₆-C₂₀ 1-alkene hydrocarbons and compositions containing the oligomers. The oligomers of EP 0 558 835 are said to be have a biodegradability of above 20% according to the CEC L-33-T-82 test method. The examples include oligomers or dimers of 1-decene, 1-dodecene and 1-tetradecene, wherein the kinematic viscosity of the oligomers and dimers are 2.0 cSt and 3.5 cSt.

In accordance with this invention there is provided a polyalphaolefin fluid which comprises an oligomer prepared from a mixture consisting essentially of C₁₂ and C₁₄ olefins, said mixture having a linear, terminal mono-olefin content of at least about 80 weight percent and a C₁₂ olefin content of from about 40 to about to about 90 weight percent with the remainder being C₁₄ olefin and with only up to about 5 weight percent of olefins having carbon numbers higher than C₁₄ or lower than C₁₂, said oligomer comprising above 50 weight percent of trimers and higher oligomers and 30 to 80 weight percent of trimers, said fluid having a kinematic viscosity of from about 5.0 to about 20mm²/s at 100°C and a biodegradability as determined by the CEC L-33 A93 test, of at least about 50 percent.

Also provided is a functional fluid or lubricant composition, said composition comprising a polyalphaolefin fluid which comprises: (a) an oligomer prepared from a mixture consisting essentially of C₁₂ and C₁₄ olefins, said mixture having a linear, terminal mono-olefin content of at least about 80 weight percent and a C₁₂ olefin content of from 40 to 90 weight percent with the remainder being C₁₄ olefin and with only up to about 5 weight percent of olefins haing carbon numbers higher than C₁₄ or lower than C₁₂, said oligomer comprising above 50 weight percent of trimers and higher oligomers and 30 to 80 weight percent of trimers, said fluid having a kinematic viscosity of from 5.0 to 20mm²/s at 100°C and a biodegradability, as determined by the CEC L-33 A93 test, of at least about 50 percent, and (b) at least one additive selected from the group consisting of dispersants, antioxidants, anti-wear agents, antifoamers, corrosion inhibitors, detergents, seal-swell agents and viscosity improvers.

C₁₂ and C₁₄ α-olefin mixtures for forming the oligomer can be prepared by mixing substantially pure 1-dodecene and 1-tetradecene. Conveniently, commercially available mixtures such as those obtained from the Ziegler chain growth process, which usually contain up to about 20 weight percent of vinylidene and internal olefins, can also be used. The mixtures may also contain up to about 5 weight percent of C₁₀ and/or C₁₆₊ olefins. Because the use of higher carbon number olefins raises the pour point of the oligomers compared to 1-decene oligomers, the proportion of C₁₄ olefin should be kept at or below about 60 weight percent, such that the mixtures contain from 40 to 90 weight percent C₁₂ olefin, preferably, from about 55 to about 80 weight percent C₁₂ olefin and more preferably, from 60 to 75 weight percent C₁₂ olefin, with the remainder being C₁₄ olefin and up to about 5 weight percent of higher or lower carbon number olefins.

The oligomers can be prepared as known in the art, preferably using a Friedel-Crafts catalyst system. The preferred Friedel-Crafts catalyst is BF₃. Pure BF₃ is not an effective oligomerization catalyst and the use of a small amount of a polar compound is necessary.

Any of the known promoters for BF₃ can be used such as water, alcohol (e.g. methanol, ethanol, n-propanol, isopropanol, n-butanol, isobutanol, n-hexanol, 2-ethyl hexanol, n-decanol, and n-dodecanol including mixtures thereof), fatty acids (e.g. valeric and capronic), organic esters (e.g. butyl acetate, methyl valerate and ethyl octanoate), ketones (e.g. methyl ethyl ketone and methyl isobutyl ketone), ethers (e.g. dibutyl ether, tetrahydrofuran and dioxane), alkoxylated alcohols (e.g. 2-ethoxy-ethanol), polyhydric alcohols (e.g. glycol and glycerol), inorganic acids (e.g. phosphoric acid), silica and zeolites.

The preferred promoters are water and alcohols containing about 1-8 carbon atoms such as methanol, ethanol, isopropanol, n-propanol, n-butanol, isobutanol, n-hexanol and n-octanol. The more preferred promoters are alcohols containing about 2-5 carbon atoms. The most preferred promoter is n-butanol.

The amount of promoter should be an amount that causes the BF₃ to act as an oligomerization catalyst. This is referred to as a promoter amount. A suitable range is 0.01-2.0 weight percent of the α-olefin, and, preferably, 0.1 to 1.0 percent.

Methods of conducting a BF₃ catalyzed oligomerization process are well known. In one mode, BF₃ is merely bubbled through a promoter-containing α-olefin reaction mixture during the oligomerization. In a preferred mode, the process is conducted under BF₃ pressure. A useful pressure is from 15 to 1000 kPa, preferably from 40 to 500 kPa and more preferably from 75 to 150 kPa.

The preferred reaction temperatures are 10 to 60°C. and more preferably 25 to 50°C. Lower temperatures will increase the amount of higher oligomers but at the cost of a slower reaction rate. High temperatures give a fast reaction rate but increased yield of dimer.

The oligomerization is usually conducted until the monomer content of the reaction mixture drops below about 5 weight percent, more preferably below about 2 weight percent. The reaction product, which is predominantly (above 50 weight percent) trimer and higher oligomers and, preferably, 30 to 80 weight percent trimer is washed to remove the BF₃ and promoter and the monomer is stripped. If desired the product can be distilled to obtain different viscosity cuts. The product fluids can by hydrogenated by conventional methods in order to improve their stability. Supported nickel catalysts are useful. For example, nickel on a Kieselguhr support gives good results. Batch or continuous processes can be used. For example, the catalyst can be added to the liquid and stirred under hydrogen pressure or the liquid may be trickled through a fixed bed of the supported catalyst under hydrogen pressures of 700 to 7,000 kPa at temperatures of at 150 to 300°C.

Fluids of the invention which have a 100°C kinematic viscosity of at least about 5 mm²/s, and especially from 5.5 to 7.5 mm²/s, are, surprisingly significantly more biodegradable than 1-decene fluids of the same viscosity range.

The fluids of the invention are useful in various functional fluid and lubricant applications and their properties can be enhanced by the use of conventional lubricant additives in total amounts of up to about 25 weight percent, and preferably from 0.1 to 20 weight percent. Such additives include, for example, dispersants, anti-oxidants, anti-wear agents, anti-foam, corrosion inhibitors, detergents, seal swell agents and viscosity index improvers. These types of additives are well known in the art. Some examples of such additives are zinc dialkyl-dithiophosphates, calcium aryl sulfonates, overbased calcium aryl sulfonates, barium phenates, barium oxide-neutralized reaction products of phosphorus pentasulfide and terpenes of high molecular weight olefins, hindered alkyl phenols, methylene-bis-dialkyl phenols, dibutyl tin sulfide, dibutyl hydrogen phosphonate, tri-cresyl-phosphate, high molecular weight alkyl succinimides of ethylene-polyamines such as tetraethylene-polyamine, sulfur-bridged alkyl phenols, sulfurized fatty acid esters and amides, silicones and dialkylesters. Proprietary combinations of such additives, "additive packages," which are tailored for specific base oils and applications, are commercially available from several sources including Ethyl Corporation. Viscosity Index (VI) improvers are separately available.

The over-all biodegradability of the formulations containing the fluids of the invention will depend upon the total functional fluid or lubricant blends including the lubricant additives.

The fluid can be used as base oils or additive oils and in blends with other oils such as, for example, C₆-C₁₀ 1-olefin based PAO's, mineral oils, synthetic esters.

Lubricant formulations of the fluids have been found to have a higher viscosity index and to exhibit a much lower tendency than 1-decene oligomer to form deposits in Panel Coker Tests.

Surprisingly, it has also been found that when heavy duty diesel engine lubricants are formulated using the fluids of the invention, a significant reduction in particular exhaust emissions occurs when compared with both mineral oil and conventional 1-decene oligomer based lubricants. For significant particulate reduction the base oil should contain at least about 50 weight percent C₁₂/C₁₄ PAO and, preferably, at least about 85 weight percent.

The invention is further illustrated by, but is not intended to be limited to, the following Examples.

### Example 1

C₁₂/C₁₄ olefin which contains about 70% C₁₂ and 30% C₁₄ by weight, with about 85 mole % being linear α-olefin, is oligomerized using a BF₃-n-butanol catalyst at a reaction temperature of about 30°C. (2.7 barg BF₃ pressure, 0.6 weight % of the monomer charge of n-butanol) for about 85 minutes cook time. The oligomer yield is about 95%. The crude product is washed, filtered and distilled to remove monomer and some dimer. The product is then hydrogenated. The product contains, by weight, about 15% dimer, 54% trimer and 31% tetramer and higher oligomers.

### Example 2

Example 1 is repeated except that the reaction temperature is about 45°C and the cook time is about 75 minutes. The crude product is distilled to remove all of the monomer and dimer, and then is hydrogenated. The product contains, by weight, about 68% trimer and 32% tetramer and higher oligomers.

The rheological properties of fluids prepared in Examples 1 and 2 were compared with those of a commercial 6 mm ²/s polyalphaolefin fluid marketed by Albemarle Corporation under the trademark, DURASYN^{™} 166.

| TEST | Ex. 1 C₁₂/C₁₄ PAO | DURASYN^{™} 166 PAO | Ex.2 C₁₂/C₁₄ PAO |
|---|---|---|---|
| K. Visc 100°C, mm²/s | 6.16 | 5.90 | 7.09 |
| K. Visc 40°C, mm²/s | 31.91 | 31.00 | 39.57 |
| Viscosity Index | 145 | 137 | 142 |
| Pour Point, °C | -39 | -69 | -39 |
| C.C.S. -30°C, mPa.s | 2233 | 2343 | 3200 |
| Flash Point, °C (PMC) | 226 | 227 | 260 |
| NOVACK, % wt loss | 7.90 | 7.00 | 4.50 |

The biodegradability of the 6 mm²/s product of Example 1 was compared to that of the DURASYN^{™} 166 PAO under the conditions of the CEC L33 A93 test.

The CEC (Coordinating European Council) L33 A93 protocol was developed to determine the persistence of 2-stroke outboard engine oil in aquatic environments. In recent years, results from this test have been applied more broadly. The test is fast becoming a standard for aquatic biodegradability for water insoluble materials. It should be noted that this test is not a test of "ready biodegradability" but "comparative biodegradability." These terms are tightly defined by regulatory bodies.

The CEC L 33 A93 test procedure is summarized as follows: Test flasks, together with poisoned flasks, (each in triplicate) containing mineral medium, test oil and inoculum are incubated for 0 to 21 days. Flasks containing calibration materials in the place of the test oil are run in parallel. At the end of the incubation times, the contents of the flasks are subjected to sonic vibration, acidified, and extracted with CCl₄ or R113. The extracts are then analyzed by Quantitative IR Spectroscopy, measuring the maximum absorption of the CH₃-band at 2930 cm⁻¹. The biodegradability is expressed as the % difference in residual oil content between the test flasks and the respective poisoned flasks at day 21.

The results are shown in Table I.

**TABLE I**

| Percentage Biodegradability (CEC L-33 A93) | | | | |
|---|---|---|---|---|
| | EMPA¹ | Ref Oil² | BfB³ | Ref Oil⁴ |
| C₁₂/C₁₄ PAO | 70.00 | 87.00 | 69.70 | 92.70 |
| DURASYN^{™}166 | | | | |
| PAO | 40.00 | 87.00 | 28.00 | 94.70 |

| | | | | |
|---|---|---|---|---|
| ¹ EMPA = Swiss Federal Laboratory for Material Testing | | | | |
| ² Ref. Oil was RL 130 | | | | |
| ³ BfB = BFB Consultants | | | | |
| ⁴ Ref Oil was RL 130 | | | | |

The 6 mm²/s PAO was also compared with the 6 mm²/s 1-decene based PAO in Panel Coker Bench Tests both as pure PAO and in SAE 5W40 heavy duty diesel oils (HDDO) formulations. The C₁₂/C₁₄ fluids, in each case, exhibited a much lower tendency to produce deposits. The results are shown in Tables II and III.

**TABLE II Panel Coker Performance - Pure PAOs Test Conditions: - Panel Temperature: 320°C Sump Oil Temperature: 140°C Test Length: 18 hrs. Air Flow: 6.30 Itr hr⁻¹ Splash Pattern: 15s splash 45s bake**

| Weight of Deposit, mg | | | | | |
|---|---|---|---|---|---|
| | Test | Test | Test | Test | |
| | No. 1 | No. 2 | No. 3 | No. 4 | Average |
| | | | | | |
| DURASYN™ 166 PAO | 509.0 | 510.0 | 528.0 | 655.0 | 551.0 |
| C₁₂/C₁₄ PAO | 120.0 | 95.00 | 155.0 | 157.0 | 132.0 |

**TABLE III Panel Coker Performance - SAE 5W40 HDDOs Test Conditions: - Panel Temperature: 360°C Sump Oil Temperature: 140°C Test Length: 18 hrs. Air Flow: 6.30 Itr hr⁻¹ Splash Pattern: 15s splash 45s bake**

| Weight of Deposit, mg | | |
|---|---|---|
| | Test No. 1 | Test No. 2 |
| SAE 5W40 (DURASYN^{™}166) PAO | 166.0 | 274.0 |
| SAE 5W40 (C₁₂/C₁₄) PAO | 50.0 | 80.0 |

### Example 3

A 5W40 heavy duty diesel oil (HDDO) was formulated using the C₁₂/C₁₄ PAO of Example 1 and compared to a reference 15W40 mineral oil based HDDO and a 5W40 HDDO formulated using a 6cSt 1-decene based oligomer fluid (Albemarle DURASYN^{™}166) for total particulate emissions. The comparison was done using a modern European heavy duty, direct injection, 6 cylinder in line 5.96 liter diesel engine turbocharged with a charge-air cooler (max power 177 kW at 2600 rpm, max torque 840 Nm) which meets Euro II European Commission Legislation emission standards (Oct. 1995). The comparison was done using the standard U.S. transient test procedure which simulates a combination of freeway and non-freeway driving with programmed acceleration, deceleration, load and no-load conditions. The formulated oil compositions are shown in Table IV. Two tests were done with each oil and the results are reported in Table V.

**Table IV TEST OIL COMPOSITIONS (By Percentage Weight)**

| COMPONENTS | TEST OIL 1 | TEST OIL 2 | TEST OIL 3 |
|---|---|---|---|
| | 15W40 | 5W40 | 5W40 |
| Mineral Base Oil | 78.50 | - | - |
| C₁₂/C₁₄ PAO | - | - | 55.42 |
| Durasyn^{™} 166 PAO | - | 55.42 | - |
| Durasyn^{™} 164 PAO | - | 6.78 | 6.78 |
| DI Package | 20.30 | 20.30 | 20.30 |
| VI Improver¹ | 0.90 | 7.50 | 7.50 |
| PPD² | 0.30 | - | - |
| Ester³ | - | 10.00 | 10.00 |
| TOTAL | 100.00 | 100.00 | 100.00 |

| | | | |
|---|---|---|---|
| NOTES: | | | |
| ¹ A non-dispersant type VI improver which was used in all three test oils which is an hydrogenated polyisoprene polymer. **Test Oil 1:** Neat VI polymer was used. **Test Oils 2 and 3:** A 15% m/m solution of VI polymer in 6cSt Polyalphaolefin was used. | | | |
| ² PPD is pour point depressant. | | | |
| ³ In test oils 2 and 3 the same ester was used - a POLYOL ester. | | | |

**TABLE V Particulate Levels HDDO**

| Oil | 1 | | 2 | | 3 | |
|---|---|---|---|---|---|---|
| Cold/Warm Engine Start | C | W | C | W | C | W |
| Particulates² Test A | 0.192 | 0.176 | 0.186 | 0.166 | 0.181 | 0.156 |
| Particulates Test B | - | 0.176 | 0.191 | 0.170 | 0.183 | 0.160 |

| | | | | | | |
|---|---|---|---|---|---|---|
| ¹ grams/horsepower/hour collected on the filter. | | | | | | |

The results, in duplicate, show a significant reduction in particulates for the C₁₂/C₁₄ PAO based lubricant.

## Claims

1. A polyalphaolefin fluid which comprises an oligomer prepared from a mixture consisting essentially of C₁₂ and C₁₄ olefins, said mixture having a linear, terminal mono-olefin content of at least about 80 weight percent and a C₁₂ olefin content of from about 40 to about 90 weight percent, with the remainder being C₁₄ olefin and with only up to about 5 weight percent of olefins having carbon numbers higher than C₁₄ or lower than C₁₂, said oligomer comprising above 50 weight percent of trimers and higher oligomers and 30 to 80 weight percent of trimers, said fluid having a kinematic viscosity of from about 5.0 to about 20 mm²/s at 100°C and a biodegradability, as determined by the CEC L-33 A93 test, of at least about 50 percent.

2. The fluid of claim 1 wherein said mixture of C₁₂ and C₁₄ olefins has a C₁₂ olefin content of from about 55 to about 80 weight percent.

3. The fluid of claim 1 wherein said mixture of C₁₂ and C₁₄ olefins has a C₁₂ olefin content of from 60 to 75 weight percent.

4. The fluid of claim 1 wherein said fluid has a kinematic viscosity of from 5.5 to 7.5 mm²/s at 100°C.

5. The fluid of claim 1 wherein said fluid has a kinematic viscosity of about 6.0 mm²/s at 100°C.

6. A functional fluid or lubricant composition, said composition comprising a polyalphaolefin fluid which comprises: (a) an oligomer prepared from a mixture consisting essentially of C₁₂ and C₁₄ olefins, said mixture having a linear, terminal, mono-olefin content of at least about 80 percent weight percent and a C₁₂ olefin content of from about 40 to about 90 weight percent with the remainder being C₁₄ olefin and with only up to about 5 weight percent of olefins having carbon numbers higher than C₁₄ or lower than C₁₂, said oligomer comprising above 50 weight percent of trimers and higher oligomers and 30 to 80 weight percent of trimers, said fluid having a kinematic viscosity of from about 5.0 to about 20 mm²/s at 100°C and a biodegradability, as determined by the CEC L-33 A93 test, of at least about 50 percent, and (b) at least one additive selected from the group consisting of dispersants, anti-oxidants, anti-wear agents, anti-foams, corrosion inhibitors, detergents, seal-swell agents and viscosity improvers.

7. The composition of claim 6 wherein the additive content is from 0.1 to 25 weight percent.

8. The composition of claim 6 wherein said mixture of C₁₂ and C₁₄ olefins has a C₁₂ olefin contest of from about 55 to about 80 weight percent.

9. The composition of claim 6 wherein said mixture of C₁₂ and C₁₄ olefins has a C₁₂ olefin content of from 60 to 75 weight percent.

10. The composition of claim 6 wherein said fluid has a kinematic viscosity of from 5.5 to 7.5mm²/s at 100°C.

11. A diesel engine lubricant which provides reduced particulate emissions comprising (a) a base oil which comprises at least 50 weight percent of an oligomer prepared from a mixture consisting essentially of C₁₂ and C₁₄ olefins, said mixture having a linear, terminal, mono-olefin content of at least about 80 weight percent and C₁₂ olefin content of from about 40 to about 90 weight percent with the remainder being C₁₄ olefin and with only up to about 5 weight percent of olefins having carbon numbers higher than C₁₄ or lower than C₁₂ said oligomer comprising above 50 weight percent of trimers and higher oligomers and 30 to 80 weight percent of trimers, said fluid having a kinematic viscosity of from about 5.0 to about 20 mm²/s at 100°C, and (b) at least one additive selected from the group consisting of dispersants, antioxidants, anti-wear agents, anti-foams, corrosion inhibitors, detergents, seal-swell agents and viscosity improvers.

12. The composition of claim 11 wherein the additive content is from 0.1 to 25 weight percent.

## Patentansprüche

1. Polyalphaolefin-Flüssigkeit, welche ein Oligomer umfaßt, das aus einer im wesentlichen aus C₁₂- und C₁₄-Olefinen bestehenden Mischung hergestellt wurde, wobei diese Mischung einen Anteil eines lineraren terminalen Mono-Olefins von mindestens etwa 80 Gew.-% und einen Anteil eines C₁₂-Olefins von etwa 40- bis etwa 90 Gew.-% aufweist, mit einer Restmenge von C₁₄-Olefin und mit lediglich bis zu etwa 5 Gew.-% an Olefinen, die Kohlenstoffzahlen von mehr als C₁₄ oder weniger als C₁₂ aufweisen, wobei das Oligomer über 50 Gew.-% an Trimeren und höheren Oligomeren und 30 bis 80 Gew.-% an Trimeren aufweist, wobei die Flüssigkeit eine kinematische Viskosität von etwa 5,0 bis etwa 20 mm²/s bei 100°C und eine biologische Abbaubarkeit von mindestens etwa 50 % aufweist, wie mit Hilfe des CEC L-33 A93 Testes bestimmt.

2. Flüssigkeit gemäß Anspruch 1, wobei die Mischung aus C₁₂- und C₁₄-Olefinen einen C₁₂-Olefin-Anteil von etwa 55 bis 80 Gew.-% aufweist.

3. Flüssigkeit gemäß Anspruch 1, wobei die Mischung aus C₁₂- und C₁₄-Olefinen einen C₁₂-Olefin-Anteil von 60 bis 75 Gew.-% aufweist.

4. Flüssigkeit gemäß Anspruch 1, wobei die Flüssigkeit eine kinematische Viskosität von 5,5 bis 7,5 mm²/s bei 100°C aufweist

5. Flüssigkeit gemäß Anspruch 1, wobei die Flüssigkeit eine kinematische Viskosität von etwa 6,0 mm²/s bei 100°C aufweist.

6. Eine funktionelle Flüssigkeits- oder Schmiermittel-Zusammensetzung, wobei die Zusammensetzung eine Polyalphaolefin-Flüssigkeit umfaßt, die umfaßt: (a) ein aus einer im wesentlichen aus C₁₂- und C₁₄-Olefinen bestehenden Mischung hergestelltes Oligomer, wobei die Mischung einen Anteil eines lineraren terminalen Mono-Olefins von mindestens etwa 80 Gew.-% und einen Anteil eines C₁₂-Olefins von etwa 40- bis etwa 90 Gew.-% aufweist, mit einer Restmenge von C₁₄-Olefin und mit lediglich bis zu etwa 5 Gew.-% an Olefinen, die Kohlenstoffzahlen von mehr als C₁₄ oder weniger als C₁₂ aufweisen, wobei das Oligomer über 50 Gew.-% an Trimeren und höheren Oligomeren und 30 bis 80 Gew.-% an Trimeren aufweist, wobei die Flüssigkeit eine kinematische Viskosität von etwa 5,0 bis etwa 20 mm²/s bei 100°C und eine biologische Abbaubarkeit von mindestens etwa 50 % aufweist, wie mit Hilfe des CEC L-33 A93 Testes bestimmt, und (b) mindestens ein Additiv, ausgewählt aus der Gruppe, bestehend aus Dispergiermitteln, Antioxidantien, Anti-Verschleißmitteln, Anti-Schaummitteln, Korrosions-Inhibitoren, Detergentien, Dicht-Schwell-Mitteln und Viskositätsverbesserern.

7. Zusammensetzung gemäß Anspruch 6, wobei der Additiv-Anteil bei 0,1 bis 25 Gew.-% liegt.

8. Zusammensetzung gemäß Anspruch 6, wobei die Mischung aus C₁₂- und C₁₄₋Olefinen einen C₁₂-Olefin-Anteil von etwa 55 bis etwa 80 Gew.-% aufweist.

9. Zusammensetzung gemäß Anspruch 6, wobei die Mischung aus C₁₂- und C₁₄₋Olefinen einen C₁₂-Olefin-Anteil von 60 bis 75 Gew.-% aufweist.

10. Zusammensetzung gemäß Anspruch 6, wobei die Flüssigkeit eine kinematische Viskosität von 5,5 bis 7,5 mm²/s bei 100°C aufweist.

11. Ein Diesel-Maschinen-Schmiermittel, welches eine verminderte Partikelemission bereitstellt, umfassend (a) ein Basisöl, welches mindestens 50 Gew.-% eines Oligomers umfasst, das aus einer Mischung hergestellt wurde, die im wesentlichen aus C₁₂- und C₁₄-Olefinen besteht, wobei die Mischung einen Anteil eines lineraren, terminalen Mono-Olefins von mindestens etwa 80 Gew.-% und einen Anteil eines C₁₂-Olefins von etwa 40- bis etwa 90 Gew.-% aufweist, mit einer Restmenge von C₁₄-Olefin und mit lediglich bis zu etwa 5 Gew.-% an Olefinen, die Kohlenstoffzahlen von mehr als C₁₄ oder weniger als C₁₂ aufweisen, wobei das Oligomer über 50 Gew.-% an Trimeren und höheren Oligomeren und 30 bis 80 Gew.-% an Trimeren aufweist, und wobei die Flüssigkeit eine kinematische Viskosität von etwa 5,0 bis etwa 20 mm²/s bei 100°C aufweist, und (b) mindestens ein Additiv, ausgewählt aus der Gruppe, bestehend aus Dispergiermitteln, Antioxidantien, Anti-Verschleißmitteln, Anti-Schaummitteln, Korrosions-Inhibitoren, Detergentien, Dicht-Schwell-Mitteln und Viskositätsverbesserern.

12. Zusammensetzung gemäß Anspruch 11, wobei der Additiv-Anteil bei 0,1 bis 25 Gew.-% liegt.

## Revendications

1. Liquide constitué de polyalphaoléfines qui comprend un oligomère préparé à partir d'un mélange consistant essentiellement en des oléfines en C₁₂ et en C₁₄, ledit mélange ayant une teneur en mono-oléfine linéaire terminale d'au moins 80 pour cent environ en poids, et une teneur en oléfine en C₁₂ d'environ 40 à environ 90 pour cent en poids, le reste étant des oléfines en C₁₄, et avec seulement jusqu'à environ 5 pour cent en poids d'oléfines ayant des nombres de carbone supérieurs à C₁₄, ou inférieurs à C₁₂, ledit oligomère comprenant plus de 50 pour cent environ en poids de trimères et d'oligomères supérieurs, et 30 à 80 pour cent en poids de trimères, ledit liquide ayant une viscosité cinématique d'environ 5,0 à environ 20 mm²/s à 100°C et une biodégradabilité, déterminée par le test CEC L-33 A93, d'au moins 50 pour cent environ.

2. Liquide selon la revendication 1, dans lequel ledit mélange d'oléfines en C₁₂ et en C₁₄, a une teneur en oléfines en C₁₂ d'environ 55 à environ 80 pour cent en poids.

3. Liquide selon la revendication 1, dans lequel ledit mélange d'oléfines en C₁₂ et en C₁₄, a une teneur en oléfines en C₁₂ de 60 à 75 pour cent en poids.

4. Liquide selon la revendication 1, dans lequel ledit liquide a une viscosité cinématique de 5,5 à 7,5 mm²/s à 100°C.

5. Liquide selon la revendication 1, dans lequel ledit liquide a une viscosité cinématique d'environ 6,0 mm²/s à 100°C

6. Liquide fonctionnel ou composition lubrifiante, ladite composition comprenant un liquide constitué de polyalphaoléfines qui comprend : (a) un oligomère préparé à partir d'un mélange consistant essentiellement en des oléfines en C₁₂ et en C₁₄, ledit mélange ayant une teneur en mono-oléfine linéaire terminale d'au moins 80 pour cent environ en poids, et une teneur en oléfine en C₁₂ d'environ 40 à environ 90 pour cent en poids, le reste étant des oléfines en C₁₄, et avec seulement jusqu'à environ 5 pour cent en poids d'oléfines ayant des nombres de carbone supérieurs à C₁₄, ou inférieurs à C₁₂, ledit oligomère comprenant plus de 50 pour cent en poids de trimères et d'oligomères supérieurs, et 30 à 80 pour cent en poids de trimères, ledit liquide ayant une viscosité cinématique d'environ 5,0 à environ 20 mm²/s à 100°C et une biodégradabilité, déterminée par le test CEC L-33 A93, d'au moins 50 pour cent environ, et (b) au moins un additif choisi au sein du groupe consistant en des dispersants, des anti-oxydants, des agents anti-usure, des anti-mousses, des inhibiteurs de corrosion, des détergents, des agents d'étanchéité et des agents améliorant la viscosité.

7. Composition selon la revendication 6, dans laquelle la teneur en additif est de 0,1 à 25 pour cent en poids.

8. Composition selon la revendication 6, dans laquelle ledit mélange d'oléfines en C₁₂ et en C₁₄, a une teneur en oléfines en C₁₂ d'environ 55 à environ 80 pour cent en poids.

9. Composition selon la revendication 6, dans laquelle ledit mélange d'oléfines en C₁₂ et en C₁₄, a une teneur en oléfines en C₁₂ de 60 à 75 pour cent en poids.

10. Composition selon la revendication 6, dans laquelle ledit liquide a une viscosité cinématique de 5,5 à 7,5 mm²/s à 100°C.

11. Lubrifiant de moteur Diesel qui procure des émissions réduites de particules, comprenant (a) une huile de base qui comprend au moins 50 pour cent en poids d'un oligomère préparé à partir d'un mélange consistant essentiellement en des oléfines en C₁₂ et en C₁₄, ledit mélange ayant une teneur en mono-oléfine linéaire terminale d'au moins 80 pour cent environ en poids, et une teneur en oléfine en C₁₂ d'environ 40 à environ 90 pour cent en poids, le reste étant des oléfines en C₁₄, et avec seulement jusqu'à environ 5 pour cent en poids d'oléfines ayant des nombres de carbone supérieurs à C₁₄, ou inférieurs à C₁₂, ledit oligomère comprenant plus de 50 pour cent en poids de trimères et d'oligomères supérieurs, et 30 à 80 pour cent en poids de trimères, ledit liquide ayant une viscosité cinématique d'environ 5,0 à environ 20 mm²/s à 100°C, et (b) au moins un additif choisi au sein du groupe consistant en des dispersants, des anti-oxydants, des agents anti-usure, des anti-mousses, des inhibiteurs de corrosion, des détergents, des agents d'étanchéité et des agents améliorant la viscosité.

12. Composition selon la revendication 11, dans laquelle la teneur en additif est de 0,1 à 25 pour cent en poids.
